# EUROPEAN PATENT APPLICATION

(11) **EP 1 666 065 A1**
(43) Date of publication of application: **07.06.2006**
(21) Application number: 04772378.8
(22) Date of filing: 23.08.2004
(51) Int. Cl.: A61K 45/00, A61K 31/7072, A61K 31/335, A61K 31/343, A61K 48/00, A61K 31/7105, A61P 29/00, A61P 37/06, A61P 19/02

(54) **THERAPEUTIC AGENT FOR AUTOIMMUNE DISEASE**

(30) Priority: 21.08.2003 JP 2003297742
(71) Applicant: Locomogene, Inc., Tokyo 105-0001 (JP)
(72) Inventor: NAKAJIMA, Toshihiro, Yokohama-shi, Kanagawa2240001 (JP); AMANO, Tetsuya, Kawasaki-shi, Kanagawa 2140005 (JP); YAMASAKI, Satoshi, Yokohama-shi, Kanagawa 2250003 (JP); YAGISHITA, Naoko, Yokohama-shi, Kanagawa 2350053 (JP)
(74) Representative: Woods, Geoffrey Corlett
(86) International application number: PCT/JP2004/012422
(87) International publication number: WO 2005/018675

(57) **Abstract**

The present invention provides an apoptosis-inducing agent or a therapeutic agent for autoimmune diseases, comprising a substance that induces ER stress; and a method for inhibiting the proliferation of cells, wherein the cells (for example, synoviocytes) are treated with the inducing agent.

SEQUENCE LISTING

<110> Locomogene, Inc.

<120> A therapeutic agent of autoimmune disease

<130> P03-0116PCT

<150> JP2003-297742
<151> 2003-09-02

<160> 2

<170> PatentIn version 3.2

<210> 1
<211> 21
<212> RNA
<213> Artificial

<220>
<223> synthetic RNA

<400> 1
cguuccuggu acgccgucau u 21

<210> 2
<211> 21
<212> RNA
<213> Artificial

<220>
<223> synthetic RNA

<400> 2
ugacggcgua ccaggaacgu u 21

## Description

### TECHNICAL FIELD

The present invention relates to a therapeutic agent for autoimmune diseases comprising a substance that induces endoplasmic reticulum stress and more particularly relates to a therapeutic agent for rheumatoid arthritis.

### BACKGROUND ART

Synoviocytes undergo abnormal proliferation in rheumatoid arthritis and play a central role in joint destruction. In order to treat rheumatoid arthritis, substantial research has therefore been carried out targeted on synoviocytes and particularly targeted on inhibiting the autonomous proliferation of these cells. However, the mechanism underlying this autonomous proliferation has not been adequately elucidated.

The present inventors have carried out immunoscreening using anti-synovial antibodies in order to search for the molecules that cause synoviocyte proliferation in rheumatoid arthritis. As a result, the inventors succeeded in isolating a protein known as "Synoviolin", which is a ubiquitin ligase present in the endoplasmic reticulum (ER), and also succeeded in cloning the gene coding for this protein.

Very interestingly, about 30% of mice that strongly expressed the Synoviolin molecule spontaneously developed arthropathy associated with synoviocyte proliferation. In contrast, syno^{+/-} mice, in which Synoviolin expression has been heteroknocked out, exhibited resistance to type II collagen-induced arthritis, which is a model for rheumatoid arthritis. It was also found that this resistance can be attributed to apoptosis increase of synoviocytes.

Based on these results, the present inventors have proposed a model in which an enhancement in the ER-associated degradation (ERAD) function, which is involved with ER stress, can cause arthropathy by triggering synoviocyte proliferation.

Synoviolin is a ubiquitin ligase (E3) with a RING finger domain and has a quality control function in the ER. The mechanism designated by ERAD, in which ER stress (described below) is attenuated by the degradation of unfolded protein that has been produced in the ER, is known to carry out this quality control task in the ER. This ERAD can be explained as follows. Protein, after its synthesis in the cytoplasm, cannot function until it has formed the correct conformation and has been transported to a specific location. Unfolded or damaged protein that has not assumed the appropriate higher order structure is checked by the quality control function possessed by the cell and is regenerated or degraded, thereby preserving homeostasis in cell function.

Protein is unstable during its biosynthesis in the lumen of the ER and as a result is vulnerable to a variety of physical-chemical stresses (for example, ischemia, hypoxia, thermal shock, amino acid deprivation, genetic mutation, and so forth). This stress is known as ER stress (ER stress) and causes an increase in the frequency of appearance in the ER of protein that has an abnormal folded structure (unfolded protein). Defective protein that has assumed an abnormal conformation is not transported out of the ER to the Golgi apparatus, and left alone the unfolded protein ends up accumulating in the ER. In response to this ER stress, the cell, using ER-specific stress response mechanisms known as UPR and ERAD, carries out degradation of the unfolded protein, thereby averting the ER stress due to the accumulation of the unfolded protein.

The present inventors, turning their attention to this ERAD mechanism, have demonstrated that inhibition of the Synoviolin-dependent ERAD mechanism is linked to the treatment of arthropathy.

However, this disease model does not necessarily also apply to human rheumatoid arthritis, and it is unclear whether the inhibition of Synoviolin expression will be effective for the treatment of human rheumatoid arthritis.

### DISCLOSURE OF THE INVENTION

An object of the present invention is to provide a drug that is effective for the treatment of autoimmune diseases and particularly rheumatoid arthritis.

As a result of intensive investigations directed to solving the aforementioned problems, the inventors, turning their attention to substances that induce ER stress, discovered that rheumatoid arthritis can be treated using such substances. The present was achieved based on this discovery.

In other words, the present invention is as follows.
(1) An apoptosis-inducing agent, comprising a substance that induces ER stress.
   Substances that induce endoplasmic reticulum stress can be exemplified by at least one selected from the group consisting of tunicamycin, thapsigargin, and brefeldin A. Inducing agent according to the present invention can further comprise siRNA against the gene that codes for Synoviolin.
(2) A therapeutic agent for autoimmune diseases, comprising a substance that induces endoplasmic reticulum stress.
   Substances that induce endoplasmic reticulum stress can be exemplified by at least one selected from the group consisting of tunicamycin, thapsigargin, and brefeldin A. Rheumatoid arthritis is an example of a target for the therapeutic agent according to the present invention. The aforementioned inducing agent can further comprise siRNA targeted to the gene that codes for Synoviolin.
(3) A method for inhibiting cell proliferation, wherein cells are treated with an inducing agent according to (1) above. The cells can be exemplified by synoviocytes.

### BRIEF DESCRIPTION OF THE DRAWINGS

Figure 1 is a photograph that shows the expression of Synoviolin in synovial membranes in rheumatoid arthritis and osteoarthritis.
Figure 2 is a photograph of Western blots that show the increased expression of Synoviolin in RA synoviocytes.
Figure 3A is a diagram that shows a reduction in proliferation activity when the expression of Synoviolin is inhibited in synoviocytes subjected to siRNA treatment.
Figure 3B is a diagram that shows the strength of apoptosis induction using tunicamycin and apoptosis induction using siRNA against Synoviolin.
Figure 4 is a graph that shows the resistance of rheumatoid synoviocytes to ER stress-induced apoptosis in comparison to other cells.
Figure 5 is a graph that shows the resistance of rheumatoid synoviocytes to the stimulation of ER stress in comparison to osteoarthritis synoviocytes.
Figure 6 is a photograph that shows ER stress in collagen-induced arthritis.

Expression of ATF6 in the knee joint of collagen-induced arthritis mice. Left: Synoviolin wild-type. Right: Synoviolin hetero knockout.

Figure 7 is a photograph that shows ER stress in rheumatoid arthritis synovial tissue.

Expression of ATF6 in rheumatoid arthritis synovial tissue. Left: tissue immunostained by anti-ATF6 antibody. Right: negative control. Upper: 100X magnification. Lower: 200X magnification.

### BEST MODE FOR CARRYING OUT THE INVENTION

The present invention is described in detail hereinbelow.

### 1. Summary

As noted above, even in the presence of ER stress, synoviocytes can proliferate due to the ERAD function.

However, there are also limits on the ERAD processing capacity, and when ER stress is further applied above this limit the ERAD function is then no longer able to contribute. Taking note of this point, the present invention, by inducing ER stress and preferably by inducing excess ER stress so as to exceed the ERAD processing capacity, succeeds in bringing about a process (inhibition of arthropathy rather than pathogenesis of arthropathy) different from the ERAD → synoviocyte proliferation → arthropathy pathogenic process.

Accordingly, the present invention characteristically checks the ERAD function and induces cell apoptosis by inducing ER stress. For example, a characteristic feature is the treatment of arthropathy by inducing apoptosis in synoviocytes, resulting in an inhibition of their proliferation.

This apoptosis denotes cell death that is caused by the cell itself and is characterized by condensation of the chromatin in the cell nucleus, fragmentation of the cell nucleus, loss of cell surface microvilli, condensation of the cytoplasm, caspase activation, loss of the mitochondrial membrane potential, and so forth. When these characteristics are produced in the cell, it is concluded that apoptosis has been induced, that is, that apoptosis has been brought about.

### 2. ER stress-inducing substances

The substance used to induce ER stress may generally be selected from substances that inhibit the function of the chaperone proteins that are necessary for the formation of the three-dimensional structure of proteins in the ER. The induction of ER stress can be confirmed from the following: activation of ER-localized transcriptional factor (ATF6: activating transcriptional factor 6), activation of ER-localized kinase (protein kinase-like ER kinase (PERK)), or activation of an ER-localized specific caspase (caspase 12).

Activation can be measured by ELISA, Western blotting, or fluorescent immunostaining techniques. As a result, substances that cause activation of the transcriptional factor, kinase, and/or caspase can be selected for the ER stress-inducing substance.

Tunicamycin, thapsigargin, brefeldin A, 2-mercaptoethanol, and so forth can be used as the ER stress-inducing substance in the present invention, and, for example, tunicamycin, thapsigargin, and brefeldin A are preferred. Tunicamycin is an antibiotic discovered as a marker of antiviral activity for the Newcastle virus and is a collection of substances in which fatty acid with a 13- to 17-member carbon chain is bonded to a β-galactosamine/α-galactosamine combination. Tunicamycin functions to selectively inhibit N-glycosidic glycosylation in the animal cell. This ER stress-inducing substance can be obtained by synthesis or can be purchased from, for example, the Sigma Company.

Thapsigargin, by inhibiting the function of chaperone molecules within the ER through an inhibitory action on the ER membrane calcium pump, can be utilized for protein inhibition in the ER and thus also as an ER stress-inducing substance.

Brefeldin A is known as a drug that inhibits intracellular protein transport, that is, transport to the Golgi apparatus. Brefeldin A inhibits the transport of secretory proteins and can induce ER stress at concentrations of 1 to 10 µg/mL.

The dosage for causing apoptosis using these ER stress-inducing substances is, for the in vitro case, 1 µg/mL to 50 mg/mL and preferably 1 µg/mL to 30 mg/mL. Or, with respect to cells the dosage is 1 µg/mL to 100 mg/mL and preferably 1 µg/mL to 50 mg/mL. When the ER stress-inducing substance is to be administered to humans, the same methods and dosages as for the therapeutic agent described below can be used.

### 3. Use of RNAi

In addition to the ER stress-inducing substances described above, RNAi (RNA interference) can also be used in the present invention in order to inhibit expression of the gene coding for Synoviolin (also referred to as the "Synoviolin gene"). RNAi is a phenomenon in which, upon the introduction of double-stranded RNA into a cell, the expression of the gene that has a sequence homologous with this RNA is inhibited.

In order to invoke RNAi and inhibit the expression of the Synoviolin gene, for example, siRNA (small interfering RNA) against the Synoviolin gene can be designed, synthesized, and deployed.

The design guidelines for the siRNA are as follows.
(a) Select a region 100 nucleotides downstream from the initiation codon of the Synoviolin-encoding gene.
(b) From the selected region, identify a sequence of 15 to 30 and preferably 19 bases that is linked to and begins at AA; a sequence is selected that has a GC content of 30 to 70% and preferably 45 to 55%.

In specific terms, the following base sequence can be used as the siRNA.
- sense strand:: CGUUCCUGGUACGCCGUCAUU (SEQ ID NO: 1)
- antisense strand::

UGACGGCGUACCAGGAACGUU (SEQ ID NO: 2)

To introduce the siRNA into the cell, for example, a method can be used in which siRNA synthesized in vitro is incorporated into plasmid DNA and this is introduced into the cell, or a method can be used in which 2 RNAs are annealed.

### 4. Method of use and dosage

Therapeutic agents of the present invention, which contain an ER stress-inducing substance as an effective component can be used against autoimmune diseases. Autoimmune diseases refers to diseases that are caused by an immune reaction against the individual's own tissue and include rheumatoid arthritis, Hashimoto's disease (chronic thyroiditis), pernicious anemia, Addison's disease, diabetes, systemic lupus erythematosus, dermatomyositis, Sjogren's syndrome, erythematosus, multiple sclerosis, myasthenia gravis, Reiter's syndrome, and Grave's disease. The therapeutic agent of the present invention can be administered orally or by a non-oral route. Examples of non-oral administration are pulmonary route dosage forms (for example, use of a nebulizer), dosage forms for administration by the nasal route, dosage forms for percutaneous administration (for example, ointments and creams), and injectable dosage forms. In the case of injectable dosage forms, systemic or local administration can be carried out by, for example, intravenous injection of e.g., a drip, intramuscular injection, intraperitoneal injection, or subcutaneous injection.

The method of administration is selected as appropriate as a function of the age and symptoms of the patient. The effective dosage is 0.1 µg to 100 mg and preferably 1 to 10 µg, in each case per 1 kg body weight per administration. However, the subject therapeutic agent is not limited to these dosages.

The therapeutic agent according to the present invention can be formulated by standard methods and may contain pharmaceutically acceptable carriers and/or excipients. These carriers and excipients can be exemplified by water, pharmaceutically acceptable organic solvents, collagen, polyvinyl alcohol, polyvinylpyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerol, glycerol, propylene glycol, petroleum jelly, paraffin, stearyl alcohol, stearic acid, human serum albumen, mannitol, sorbitol, lactose, and surfactants acceptable as pharmaceutical excipients.

A single excipient or a suitable combination of excipients is selected from the preceding in correspondence to the dosage form of the therapeutic agent according to the present invention. For example, when an injectable formulation is employed, this can be prepared by dissolving the purified ER stress-inducing substance in a solvent (for example, physiological saline, buffer, glucose solution) and adding thereto, for example, Tween 80, Tween 20, gelatin, or human serum albumen. Or, a dosage form requiring dissolution before use can be prepared by freeze drying. For example, sugar alcohols or sugars, such as mannitol or glucose, can be used as the bulking agent for freeze drying.

The dosage in the case of the admixture of siRNA is 0.01 to 10 µg/mL and preferably 0.1 to 1 µg/mL.

The present invention is described more specifically hereinbelow through examples. However, the present invention is not limited to these examples.

### Example 1

In order to confirm whether the expression of Synoviolin is increased in synovial tissue in rheumatoid arthritis, an investigation was carried out for this example of synovial tissue from 10 patients with rheumatoid arthritis and 5 patients with osteoarthritis using an immunostaining protocol with anti-Synoviolin monoclonal antibody.

This protocol is described below.

Paraformaldehyde-fixed tissue was embedded in paraffin, thin-sectioned to a thickness of 4 micrometers, and bonded to a glass slide. The paraffin was removed from the bonded section using xylene and the endogenous peroxidase was deactivated by permeation at room temperature in 3% aqueous hydrogen peroxide in methanol. After washing with phosphate buffer, nonspecific reactions were inhibited with the blocking reagent contained in the VECTASTAIN® ABC (Peroxidase) kit from the Vecstatin Co., and the section was then reacted for 1 hour at room temperature with anti-Synoviolin monoclonal antibody diluted to 1 µg/mL with phosphate buffer. After this, reaction was carried out according to the manufacturer's instructions at room temperature with the peroxidase conjugate contained in the VECTASTAIN® ABC (Peroxidase) kit, and color development was carried out for 10 minutes at room temperature using a DAB substrate from the Sigma Co. Staining was completed by carrying out nuclear staining with methyl green as a counterstain.

Western blotting was carried out as follows. 300,000 cultured synoviocytes were seeded to a 60-mm culture dish, and the protein was extracted after 24 hours using a protein extraction buffer containing 50 mM Tris base, 150 mM NaCl, 0.1 SDS, 1%, and 1 µg/mL PMSF. To this was added SDS sample buffer, and, after denaturation for 5 minutes at 100°C, separation was carried out on 10% acrylamide gel. The separated protein was transferred for 2 hours with a 250 mA current to a nitrocellulose membrane. The membrane was blocked for 1 hour at room temperature by 5% skim milk in TBST. An antigen/antibody reaction was carried out on this membrane for 1 hour at room temperature using anti-Synoviolin diluted to 0.1 µg/mL using TBST prepared with 0.5% skim milk. After washing with TBST, reaction with anti-mouse HRP antibody, a secondary antibody, was carried out; detection was carried out by chemofluorescence visualization.

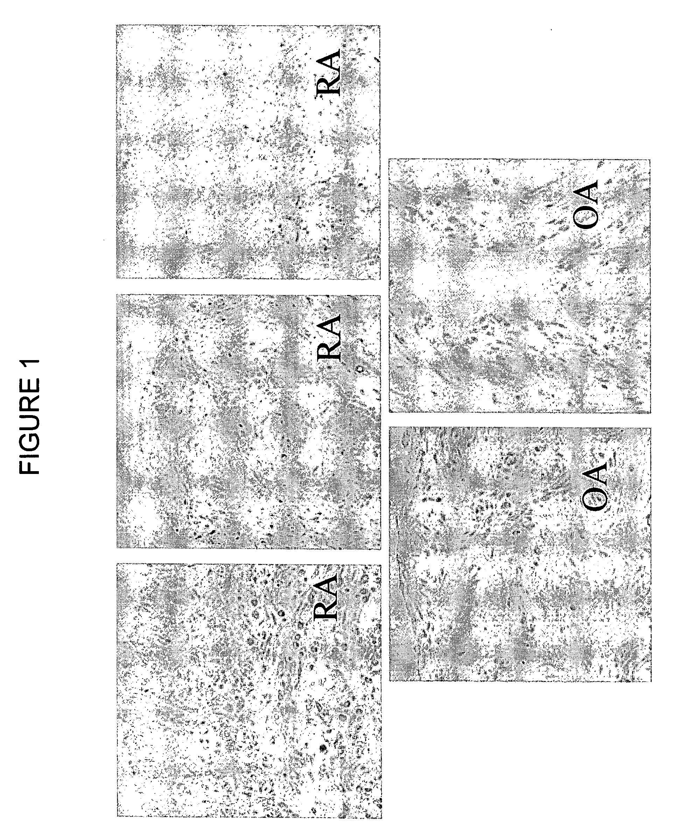

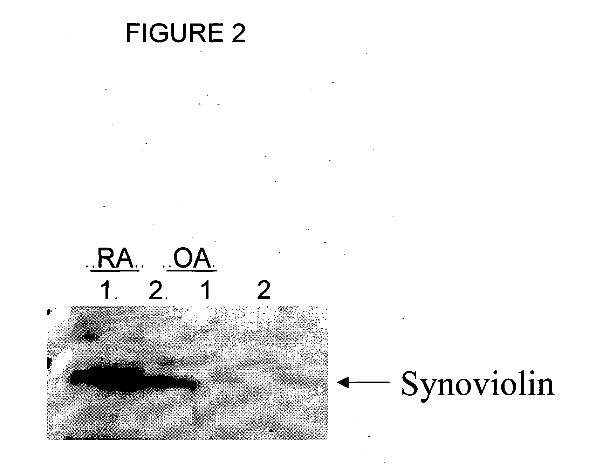

The results demonstrated that Synoviolin expression in synoviocytes from rheumatoid arthritis synovial membranes was very substantially increased over that for osteoarthritis synovial membranes (Figure 1), i.e., the results demonstrated the presence of a Synoviolin-dependent ERAD enhancement. Figure 1 demonstrates the stronger expression of Synoviolin in rheumatoid arthritis synovial membrane tissue (RA: upper row) than in osteoarthritis synovial membrane tissue (OA: lower row). The elevated expression of Synoviolin in RA synoviocytes was also confirmed by Western blotting using cultured cells (Figure 2).

### Example 2

Example 1 showed that the expression of Synoviolin is substantially increased in synoviocytes in the rheumatoid arthritis synovial membrane. However, it is not clear whether synoviocyte proliferation in human rheumatoid arthritis can be inhibited by inhibiting the augmented Synoviolin-dependent ERAD function seen in rheumatoid arthritis.

Apoptosis through the induction of ER stress was therefore investigated using tunicamycin, an ER-inducing agent. Investigations were also carried out at this time using synoviocytes treated with small interfering RNA (siRNA) against the Synoviolin gene.

That is, a test was carried out to confirm the possibility of deactivating synoviocytes and inducing apoptosis through an artificial inhibition of Synoviolin, whose expression is increased in the rheumatoid arthritis synovial membrane, by treatment with siRNA.

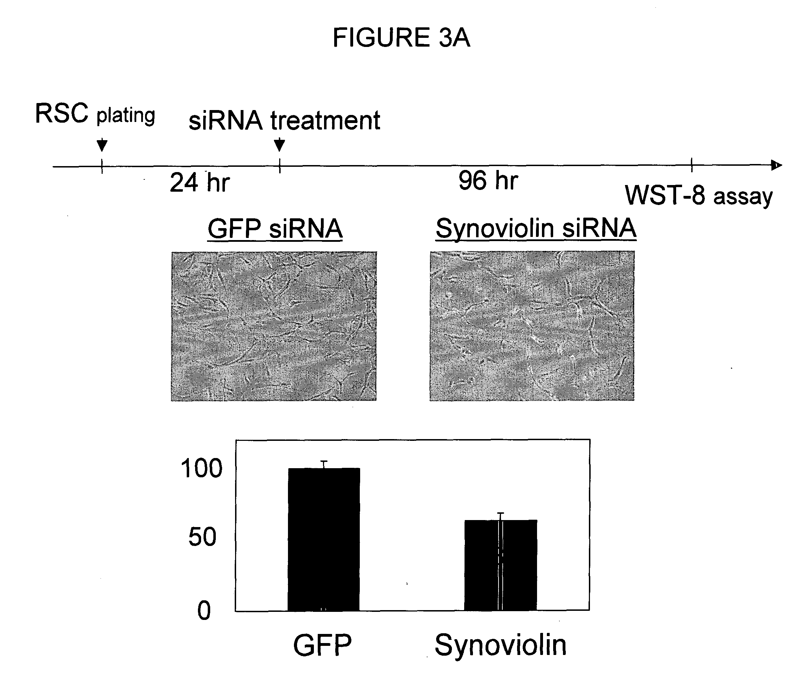

The experiments were carried out as follows. Rheumatoid synoviocytes were seeded at 160 each into a 96-well, flat-bottom plate from the Falcon Co. After 24 hours, treatment was carried with anti-Synoviolin siRNA or anti-GFP siRNA, and the proliferation activity was investigated after an additional 96 hours. The GFP was used in this case as a negative control. The degree of cell proliferation was measured using a WST-8 assay from Dojindo Laboratories. It was demonstrated that the proliferation activity of rheumatoid synoviocytes was reduced to about 60% (GFP = 100) by siRNA-mediated inhibition of Synoviolin expression in rheumatoid synoviocytes (Figure 3A).

Then, synoviocytes were seeded at 2500 each to an eight-well Lab-Tek Chamber from the Nunc Company and were incubated for 24 hours in an incubator under 5% CO₂ at 37°C. 67 nM siRNA was thereafter introduced into the synoviocytes using TransIT-TKO transfection reagent from the Miru Bio Corporation. After additional incubation for 48 hours in an incubator under 5% CO₂ at 37°C, 50 µg/mL tunicamycin was added, followed by incubation for 48 hours under the same conditions. The cells were then fixed and nuclear condensation was confirmed by DNA staining with Hoechst 33258.

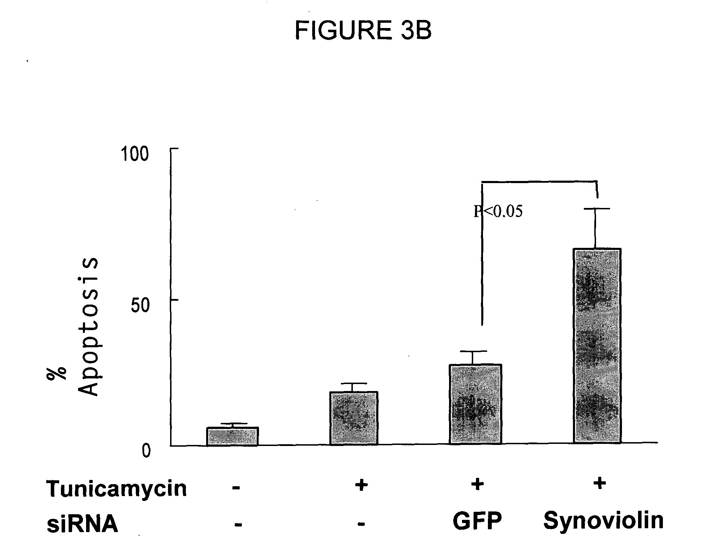

The results show that tunicamycin caused apoptosis induction in synoviocytes and, furthermore, that apoptosis induction in synoviocytes was increased by the co-use of siRNA (Figure 3B).

The preceding results show that the model for arthropathy pathogenesis demonstrated by the inventors with a mouse model, i.e., that an augmentation of Synoviolin-dependent ERAD triggers arthropathy via the proliferation of synoviocytes due to evasion of apoptosis while, conversely, synovial membrane proliferation can be inhibited by invoking apoptosis by means of Synoviolin inhibition, also applies to human synoviocytes.

The human rheumatoid arthritis synovial membrane is resistant to ER stress, and, by activating this signal, it is possible to inhibit the proliferation of synoviocytes and also to induce apoptosis.

### Example 3

This example examined the ER stress-induced apoptosis of rheumatoid synoviocytes using ER stress-inducing agents, in relation to whether or not synoviocytes and particularly rheumatoid synoviocytes exhibit resistance to ER stress-induced apoptosis.

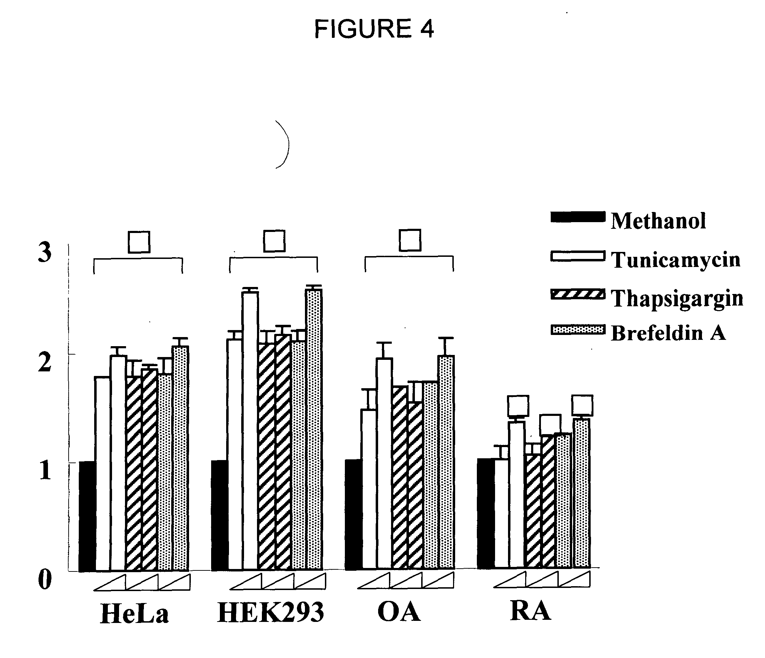

The experiments were carried out as follows. Rheumatoid synoviocytes, osteoarthritis synoviocytes, HeLa cells, and HEK293 cells were seeded at 3000 each into a 96-well, flat-bottom plate from the Falcon Co. After 24 hours, ER stress-induced apoptosis was induced by treatment for 48 hours with the following ER stress-inducing agents: tunicamycin (10 or 100 µg/mL), thapsigargin (1 or 10 µM), or brefeldin A (10 or 100 µg/mL). For each cell type, the degree of apoptosis induction was measured using the ssDNA Apoptosis ELISA kit from Chemicon International, Inc. It was shown that synoviocytes and particularly rheumatoid synoviocytes had resistance to ER-induced apoptosis (Figure 4).

### Example 4

Using the ER stress-inducing agent tunicamycin, this example examined whether or not rheumatoid synoviocytes exhibit resistance to the stimulation of ER stress.

The experiments were carried out as follows. Whether human rheumatoid synoviocytes exhibit resistance to ER stress was investigated by treating rheumatoid synoviocytes (RA: 5 cases) and osteoarthritis synoviocytes (OA: 5 cases) for 24 hours with the ER stress-inducing agent tunicamycin. Endoplasmic reticulum stress-inducible apoptosis was induced using tunicamycin at concentrations of 10,30, and 100 µg/mL. Apoptosis was quantitated using the ssDNA Apoptosis ELISA kit from Chemicon International, Inc.

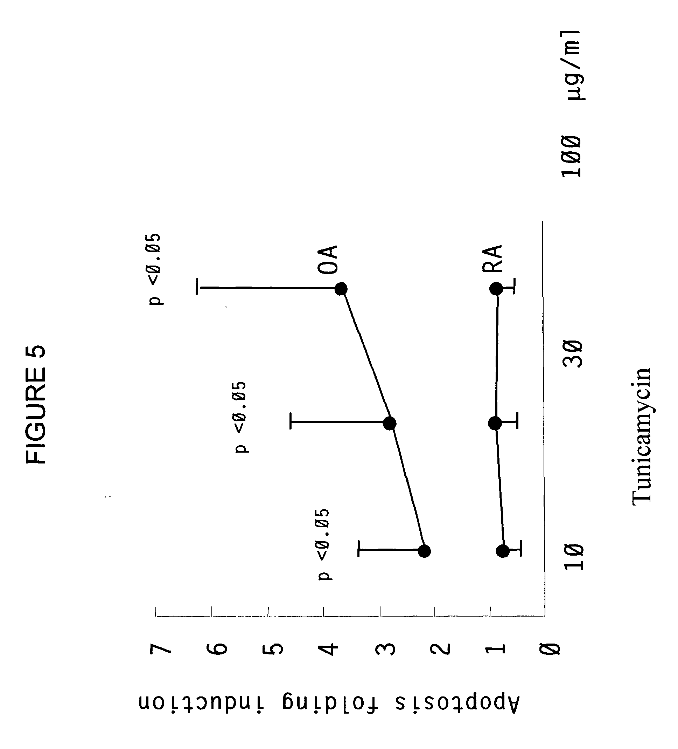

It was found as a result that, in comparison to osteoarthritis synoviocytes, rheumatoid synoviocytes are resistant to ER stress-inducible apoptosis. (10 µg/mL: RA 0.73 ± 0.30, OA 2.18 ± 1.19; 30 µg/mL: RA 0.84 ± 0.35, OA 2.76 ± 1.84; 100 µg/mL: RA 0.81 ± 0.28, OA 3.65 ± 2.53) (Figure 5).

### Example 5

Using the knee joint of Synoviolin wild-type and Synoviolin-deficient mice in which collagen-induced arthritis has been induced, this example investigated whether or not ER stress is present in collagen-induced arthritis.

That is, the presence/absence of ATF6 expression was determined by tissue immunostaining in Synoviolin wild-type and Synoviolin-deficient mice in which collagen-induced arthritis had been induced.

The experiments were carried out as follows. The knee joint was removed from Synoviolin wild-type and Synoviolin-deficient mice in which collagen-induced arthritis had been induced. After fixing for 4 hours with a 4% paraformaldehyde solution, the paraffm-embedded specimen was thin-sectioned to a thickness of 4 micrometers and was bonded on a glass slide. After a paraffin removal step, the expression of activating transcriptionalal factor 6 (ATF6), which accumulates in the cell nucleus in the presence of ER stress, was investigated. Reaction was carried out, in each case at a concentration of 0.001 mg/mL, with anti-ATF6 goat polyclonal antibody (Santa Cruz Biotechnology, Inc.) and normal goat IgG (Dako Co.), and tissue immunostaining was carried out using a Vectastain kit from Vector Laboratories and the diaminobenzidine chromogenic system.

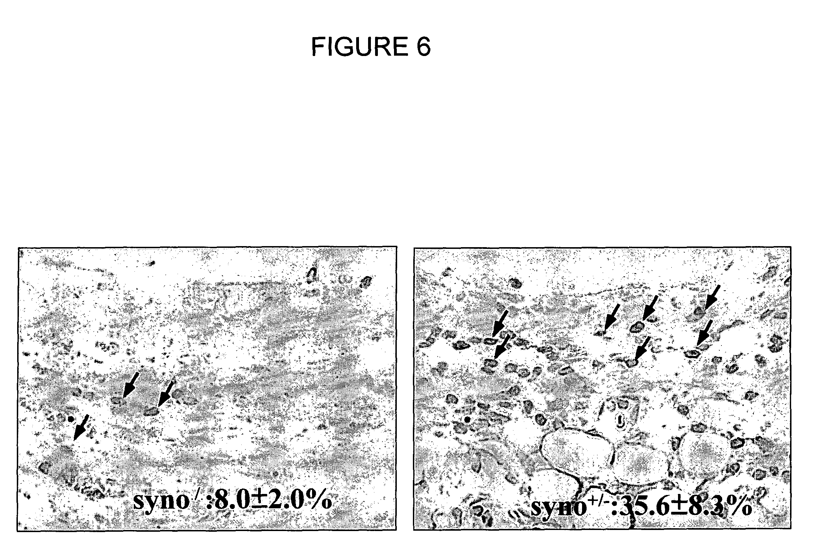

As shown by the arrows in Figure 6, it was demonstrated that ATF6 accumulation in the cell nucleus, that is, ER stress, was invoked by collagen-induced arthritis. In addition, the fact that this phenomenon was quite substantial in the Synoviolin-deficient mice demonstrated that Synoviolin has the ability to inhibit the ER stress caused by arthritis (Figure 6).

### Example 6

It was shown in Example 5 that Synoviolin has the ability to inhibit the ER stress caused by arthritis. In addition, investigations with mice have made it clear that an augmentation of ERAD is related to the proliferation of synoviocytes. However, it remains unclear as to whether ERAD augmentation can also act as an effective mechanism for synoviocyte proliferation in human rheumatoid arthritis synovial tissue.

Therefore, the presence of ER stress in human rheumatoid arthritis synovial tissue was confirmed with the objective of investigating whether ERAD augmentation can also act as an effective mechanism for synoviocyte proliferation in human rheumatoid arthritis synovial tissue. If the presence of ER stress can be shown, ERAD augmentation, as a mechanism for evading this, will therefore have critical significance for the proliferation of synoviocytes in rheumatoid arthritis.

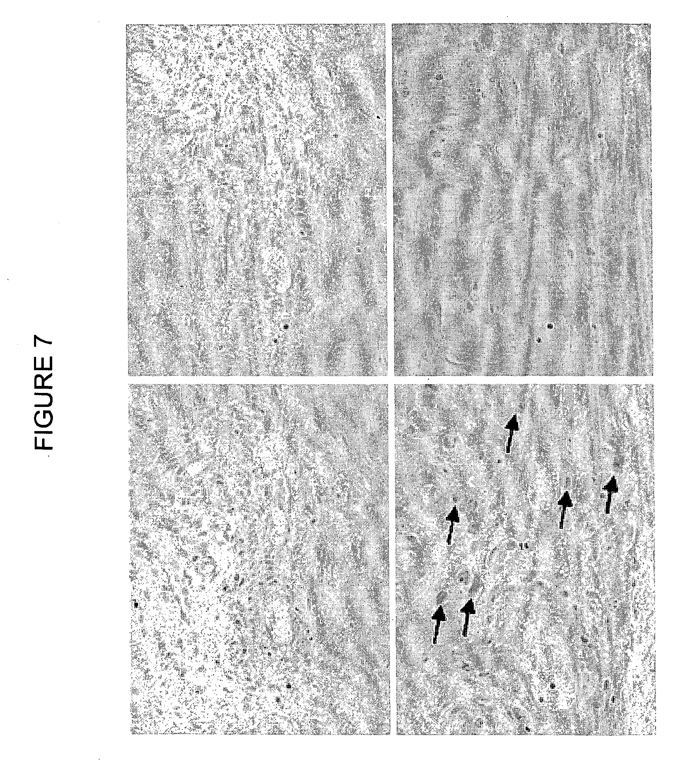

The experiments were carried out as follows. The human synovial tissue removed from rheumatoid arthritis patients during knee replacement surgery was recovered after obtaining written consent. After fixing for 4 hours with a 4% paraformaldehyde solution, the paraffin-embedded specimen was thin-sectioned to a thickness of 4 micrometers and was bonded on a glass slide. After a paraffin removal step, the expression of activating transcriptionalal factor 6 (ATF6), which accumulates in the cell nucleus in the presence of ER stress, was investigated. Reaction was carried out, in each case at a concentration of 0.001 mg/mL, with anti-ATF6 goat polyclonal antibody (Santa Cruz Biotechnology, Inc.) and normal goat IgG (Dako Co.), and tissue immunostaining was carried out using a Vectastain kit from Vector Laboratories and the diaminobenzidine chromogenic system. As a result, and as shown by the arrows in Figure 7, accumulation of ATF6 in the cell nucleus was seen in the rheumatoid arthritis synovial tissue. This confirmed the presence of ER stress (Figure 7).

### INDUSTRIAL APPLICABILITY

A therapeutic agent for autoimmune diseases is provided by the present invention. The therapeutic agent according to the present invention is useful, for example, as a therapeutic drug for rheumatoid arthritis.

### SEQUENCE LISTING FREE TEXT

SEQ ID NO: 1: synthetic RNA
SEQ ID NO:2: synthetic RNA

## Claims

1. An apoptosis-inducing agent, comprising a substance that induces endoplasmic reticulum stress.

2. The inducing agent according to claim 1, wherein the substance that induces endoplasmic reticulum stress is at least one selected from the group consisting of tunicamycin, thapsigargin, and brefeldin A.

3. The inducing agent according to claim 1 or 2, further comprising siRNA against the gene that codes for Synoviolin.

4. A therapeutic agent for autoimmune diseases, comprising a substance that induces endoplasmic reticulum stress.

5. The therapeutic agent according to claim 4, wherein the substance that induces endoplasmic reticulum stress is at least one selected from the group consisting of tunicamycin, thapsigargin, and brefeldin A.

6. The therapeutic agent according to claim 4, wherein the autoimmune disease is rheumatoid arthritis.

7. The therapeutic agent according to any of claims 4 to 6, further comprising siRNA against the gene that codes for Synoviolin.

8. A method for inhibiting the proliferation of cells, wherein the cells are treated with an inducing agent according to any of claims 1 to 3.

9. The method according to claim 8, wherein the cells are synoviocytes.
